# EUROPÄISCHE PATENTANMELDUNG

(11) **EP 4 339 182 A1**
(43) Veröffentlichungstag der Anmeldung: **20.03.2024**
(21) Anmeldenummer: 23193919.0
(22) Anmeldetag: 29.08.2023
(51) Int. Cl.: C07C 51/43, C07C 53/08

(54) **VERFAHREN ZUR ANREICHERUNG VON ESSIGSÄURE AUS WÄSSRIGEN ESSIGSÄUREMISCHUNGEN, DIE ANDERE NIEDERE CARBONSÄUREN ENTHALTEN**

(30) Priorität: 13.09.2022 DE 102022123269
(71) Anmelder: ProFagus GmbH, 37194 Bodenfelde (DE)
(72) Erfinder: Schwiderski, Martin, 37154 Northeim (DE); Wosnitza, Bernward, 37124 Rosdorf (DE); Höfer, Frank, 67098 Bad Dürkheim (DE)
(74) Vertreter: karo IP

(57) **Zusammenfassung**

Die Erfindung betrifft ein Verfahren zur Anreicherung von Essigsäure aus wässrigen Essigsäuremischungen, die andere niedere Carbonsäuren wie Ameisensäure, Propionsäure und Buttersäure enthalten, durch Kristallisation.

## Beschreibung

Die vorliegende Erfindung betrifft ein Verfahren zur Anreicherung von Essigsäure aus wässrigen Essigsäuremischungen, die andere niedere Carbonsäuren wie Ameisensäure, Propionsäure und Buttersäure enthalten, durch Kristallisation.

Essigsäure ist heute eines der wesentlichen Grundprodukte der chemischen Industrie. Sie dient als Rohstoff für viele weitere Produkte und Wertschöpfungsketten (Vinylacetat, Essigsäureanhydrid, Celluloseacetat, Farben und Klebstoffe, Essigsäureester als Lösungsmittel).

Es existiert eine Vielzahl von Herstellverfahren für Essigsäure:
- Fermentation von Wein
- Isolation von Essigsäure aus dem während der Holzverkohlung anfallenden Holzessig
- Oxidation von Acetaldehyd (Wacker-Verfahren)
- Oxidation von n-Butan
- Carbonylierung von Methanol (Monsanto-Verfahren)

### Holzverkohlung

Holzessig ist eine verdünnte wässrige Essigsäurelösung mit anderen Nebenkomponenten. Diese kann destillativ nur mit sehr hohem Aufwand in eine wasserfreie Qualität überführt werden. Deswegen konzentrieren sich die Verfahren zur Gewinnung wasserfreier Essigsäure, die in der ersten Auflage des Ullmann's 1914 (Ullmann Enzyklopädie der Technischen Chemie) beschrieben sind, auf die Freisetzung aus den Acetatsalzen durch Mineralsäuren, vorzugsweise mit Schwefelsäure. Das wichtigste Rohmaterial war der durch trockene Destillation von Holz gewonnene Holzessig, der kostengünstiger als Gärungsessig zur Verfügung stand. Durch Umsetzung mit Kalk wurde Graukalk gewonnen, der bis zu 83 % Calciumacetat enthielt. Die daraus durch Säurebehandlung erhaltene Rohessigsäure musste anschließend durch Rektifikation von den Nebenprodukten der Holzpyrolyse befreit werden. Seinerzeit wurden in Deutschland jährlich rund 35.000 t essigsaurer Kalk auf Essigsäure und ihre Derivate aufgearbeitet.

### Oxidation von Acetaldehyd

Während in den beiden ersten Ullmanns-Auflagen die Oxidation von Acetaldehyd zu Essigsäure lediglich als Möglichkeit für zukünftige Anlagen beschrieben wurde, ist dieser Weg in der dritten Auflage bereits der dominierende. Zu diesem Zeitpunkt (1953) spielte der Holzessig praktisch keine Rolle mehr, und nach dem Gärverfahren wurden nur rund 10 % gewonnen (bezogen auf 100%ige Essigsäure). Die Oxidation von Acetaldehyd gelingt mit hoher Selektivität über Peressigsäure als Zwischenstufe. Die Reaktion wird bei 50 - 70 °C in Blasensäulenreaktoren in Essigsäure durchgeführt, wobei Katalysatoren wie Manganacetat zur Zersetzung der Peressigsäure eingesetzt werden. Große Anlagen wurden beispielsweise von den Farbwerken Hoechst und der Celanese Corp. betrieben. Bis Ende des 2. Weltkriegs wurde Acetaldehyd hauptsächlich durch die Quecksilber-katalysierte Wasseranlagerung an Acetylen hergestellt.

Da Acetylen über die Zwischenstufe Calciumcarbid aus gebranntem Kalk und Koks in den elektrisch beheizten Carbidöfen gewonnen wurde, war dies ein klassisches Beispiel einer Kohle-basierten Chemie. Mit dem Aufschwung der Petrolchemie nach dem 2. Weltkrieg wurde diese Synthese vollständig durch die Oxidation von Ethylen nach dem Wacker-Hoechst-Verfahren verdrängt. Die Reaktion von Ethylen mit (Luft-)Sauerstoff erfolgt in einem Blasensäulenreaktor durch den Redox-Katalysator CuCl₂/PdCl₂. Dabei oxidiert das Palladium(II)chlorid Ethylen zu Acetaldehyd unter Bildung von metallischem Palladium(0). Dessen Re-Oxidation erfolgt durch Kupfer(II)chlorid, das seinerseits mit (Luft-)Sauerstoff regeneriert wird. Die Herstellung von Acetaldehyd ist ebenfalls durch die katalytische Dehydrierung von Ethanol in der Gasphase möglich. Dieser Weg war in der Vergangenheit wegen der zu hohen Kosten für Ethanol unwirtschaftlich, könnte aber in Zukunft wegen der besseren Verfügbarkeit von Bioethanol eine Rolle spielen.

### Oxidation von n-Butan

Bereits seit 1884 war die Direktoxidation von Butan oder Naphtha zu Essigsäure als Hauptprodukt bekannt. Mit dem starken Ausbau der Erdölverarbeitung fielen in den Raffinerien gasförmige Kohlenwasserstoffe an, die zunächst weder für Kraftstoffe noch in der chemischen Industrie verwendet wurden. Daher wurde ab 1952 die Butanoxidation von Firmen wie Union Carbide Corp., Celanese Corp. und den Chemischen Werken Hüls in Anlagen mit Essigsäurekapazitäten von bis zu 300.000 t/a genutzt. Die Reaktion verläuft in der Flüssigphase über Peroxyradikale bei 130 - 200 °C unter Druck von 60 - 80 bar, wobei Essigsäure als Lösemittel unter vollständigem Sauerstoffumsatz verwendet wird. Die Aufarbeitung des Reaktoraustrags ist komplex, da neben Essig- auch Ameisen-, Propion- und Buttersäure sowie neutrale Produkte wie Ketone, Aldehyde, Ester und Alkohole entstehen.

Nachteilig für die Investitionskosten ist der Zwang zur Verwendung teurer, korrosionsfester Materialien. Wegen der stark gestiegenen Energiepreise ist dieser Herstellweg nicht mehr wirtschaftlich und der Betrieb dieser Anlagen wurde eingestellt.

### Carbonylierung von Methanol

Die Carbonylierung von Methanol ist heute der bevorzugte Weg zur Herstellung von Essigsäure und wird in praktisch allen Neuanlagen der letzten 30 Jahren genutzt. Hierfür gibt es zwei Gründe: neben der sehr hohen Selektivität dieser Synthese sind dies die gute Verfügbarkeit und niedrigen Kosten der Rohstoffe. Auch die Methanolcarbonylierung hat mehrere Innovationszyklen durchlaufen. Grundsätzlich seit 1913 bekannt, ergaben schließlich die systematischen Arbeiten von Reppe und Mitarbeitern 1941 technisch anwendbare Katalysatoren auf Basis von Metallcarbonylen. Wegen der drastischen Reaktionsbedingungen und der korrosiven Reaktionsmischung konnte aber erst 1960 in Ludwigshafen eine technische Anlage in Betrieb genommen werden, nachdem entsprechende Legierungen zur Verfügung standen. Dieses BASF-Hochdruckverfahren arbeitete bei 250 °C und 700 bar mit einem homogenen Katalysator auf Basis von Cobaltiodid. Trotz dieser Pionierleistung konnte sich dieses Verfahren nicht durchsetzen, auch wenn diese Anlage stufenweise auf eine Kapazität von zuletzt rund 45.000 t/a erweitert und eine zweite Anlage in Lizenz betrieben wurde. Denn nur zehn Jahre nach Inbetriebnahme des BASF-Verfahrens nahm die Monsanto Corp. eine Essigsäureanlage nach einem neuen Niederdruckverfahren mit einer Kapazität von 135.000 t/a in Betrieb.

Basierend auf den Fortschritten der homogenen Katalyse mit Edelmetallkomplexen wurde ein Katalysator auf Basis löslicher Rhodiumverbindungen und von Methyliodid entwickelt, das bereits bei 180 °C und einem Druck von nur 30 bar hochaktiv war. Gleichzeitig erreichte dieser Katalysator eine beeindruckende Essigsäure-Selektivität von rund 99 % bezüglich Methanol und rund 90 % bezüglich Kohlenmonoxid. Das Niederdruckverfahren war entsprechend sowohl bei den Rohstoff- als auch bei den investitionsbedingten Fixkosten deutlich günstiger und konnte sich so weltweit durchsetzen.

Das Monsanto-Essigsäureverfahren ist ein herausragendes Beispiel für die Leistungsfähigkeit der homogenen Katalyse und fehlt in keinem einschlägigen Lehrbuch. Erster Lizenznehmer des Verfahrens war die Celanese Chem. Comp. in einer Anlage in Clear Lake, Texas, mit einer Kapazität von 270.000 t/a. Celanese entwickelte Anfang der 1980er das Verfahren unter anderem durch Modifikation des Katalysators durch anorganische Iodide weiter, wodurch die Reaktion bei niedrigen Wasserkonzentrationen durchgeführt werden konnte. Dieser neue Prozess erlaubte bei vergleichsweise geringen Investitionen die stufenweise Erweiterung der Anlage auf die dreifache Kapazität. Infolge der Neuorientierung der Monsanto wurden die Rechte an diesem Verfahren 1986 an BP Chemicals verkauft. Auch BP entwickelte das Niederdruckverfahren durch Verwendung von homogenen Iridium-/Methyliodidkatalysatoren weiter. Dadurch war es ebenfalls möglich, die Synthese bei niedrigen Wasserkonzentrationen mit sehr hohen Raum-Zeit-Ausbeuten zu betreiben.

Heutige "World-scale"-Essigsäure-Anlagen von den Technologie- und Weltmarktführern BP oder Celanese besitzen Kapazitäten von rund 1 Mio. t./a. Auch wenn die heutigen Essigsäureverfahren eine beeindruckende Effizienz erreicht haben, besteht Potential für weitere Innovationen. So hat BP Chemicals kürzlich die Einführung einer neuen Technologie angekündigt, die auf der Carbonylierung von Dimethylether zu Methylacetat beruht. Laut Patentliteratur wird dabei ein nicht-korrosiver, heterogener Zeolith-Katalysator eingesetzt.

Bei allen Herstellverfahren von Essigsäure entstehen unerwünschte Koppelprodukte. Bei der Fermentation von Glucose fallen Buttersäure und Milchsäure an. Die Oxidation von Butan liefert neben kurzkettigen Alkoholen und Estern Ameisensäure und Buttersäure als Nebenprodukte. Ameisensäure fällt auch bei der Oxidation von Acetaldehyd an. Im Monsanto-Prozess kommt es durch Ethanol-Verunreinigungen im Methanol während der Carbonylierung zur Produktion der unerwünschten Propionsäure.

In allen o. g. Verfahren muss die Essigsäure daher durch geeignete Verfahren von den verunreinigenden Komponenten befreit werden.

Eine destillative Trennung von wässrigen Essigsäure-Ameisensäure-Mischungen führt aufgrund des binären Ameisensäure-Wasser-Maximumazeotrops sowie eines zusätzlich auftretenden ternären Essigsäure-Ameisensäure-Wasser-Azeotrops nur zu limitierten Ausbeuten an Essigsäure. Außerdem muss aufgrund der nahen beieinander liegenden Siedepunkte mit hohen Betriebs- und Investitionskosten für eine solche Trennaufgabe gerechnet werden.

In der US3660483A wird ein Verfahren beschrieben, in dem die Ameisensäure von Essigsäure mittels Reaktivrektifikation entfernt wird. Dazu wird Methanol mit Ameisensäure umgesetzt und der entstandene Ameisensäuremethylester kann leicht destillativ von Essigsäure getrennt werden.

In der US3801629A werden Wasser und Ameisensäure aus einer essigsauren Mischung ebenso mittels Reaktivrektifikation entfernt. Hier werden anstelle von Methanol n-Butanol bzw. Isoamylalkohol verwendet.

In der WO02/053524A2 wird ein Gemisch aus Essigsäure, Ameisensäure und Wasser zunächst mit Diisopropylether entwässert und anschließend die Ameisensäure unter Zuhilfenahme von Cyclopentan destillativ entfernt.

Die beschriebenen Verfahren weisen alle einige Nachteile auf. Der durch die Destillation auftretende Essigsäuredampf führt an den Anlagenteilen zu Korrosion und somit zu einem erhöhten Wartungsaufwand. Von einer Verwendung von leicht entflammbaren, explosionsgefährlichen und gesundheitsschädlichen Lösungsmitteln sollte aus sicherheits- und gesundheitstechnischen Aspekten abgesehen werden. Keines der beschriebenen Verfahren trennt sowohl die im Vergleich zur Essigsäure leichtsiedenden Komponenten Wasser und Ameisensäure als auch die schwersiedenden Komponenten Propion- und Buttersäure in einer Verfahrensstufe ab. Grundsätzlich sind die destillativen Trennaufgaben mit hohem Apparateaufwand, hohem Energieeinsatz und komplexen Steuerungen verbunden.

Die vorliegende Erfindung wurde vor dem Hintergrund des vorstehend beschriebenen Standes der Technik gemacht, wobei es die Aufgabe der Erfindung war, ein Verfahren zu entwickeln, welches die Konzentrationen sowohl an den Leichtsiedern Wasser und Ameisensäure als auch an den Schwersiedern Propion- und Buttersäure in einem Verfahrensschritt abreichert (und entsprechend die Konzentration an Essigsäure anreichert). Die Temperatur soll aufgrund von Korrosionsproblemen moderat gehalten werden. Auf die Verwendung von Lösungsmitteln soll verzichtet werden.

Die Erfindung betrifft ein Verfahren zur Anreicherung von Essigsäure aus wässrigen Essigsäuremischungen, die mindestens 50 Gew.-% Essigsäure und als Nebenbestandteile Ameisensäure, Propionsäure und/oder Buttersäure enthalten, bei dem
(a) eine wässrige Essigsäuremischung durch Abkühlen auf eine Temperatur von 1 bis 25 °C unterhalb der konzentrationsabhängigen Liquidustemperatur kristallisiert wird, und
(b) die gebildete kristalline Phase von der flüssigen Phase mechanisch abgetrennt wird, oder
(c) die gebildete kristalline Phase durch graduelles Erwärmen zum Teil wieder aufgeschmolzen und die verbleibende kristalline Phase anschließend mechanisch abgetrennt wird.

Dem Fachmann ist klar, dass prinzipiell auch auf Temperaturen unterhalb 25 °C der konzentrationsabhängigen Liquidustemperatur abgekühlt werden kann. Physikalische Gründe stünden dem nicht entgegen. Beispielsweise könnte man auch auf 50 °C oder 100 °C unterhalb der Liquidustemperatur abkühlen. Dadurch würden die wässrigen Essigsäuremischungen aber vollständig gefrieren (kristallisieren), so dass anschließend wieder auf 1 bis 25 °C unterhalb der Liquidtemperatur erwärmt werden müsste, damit nach dem erfindungsgemäßen Verfahren neben einer kristallinen Phase (in der Essigsäure angereichert ist) auch wieder eine flüssige Phase (in der die abzutrennenden Nebenkomponenten angereichert sind) vorliegt, aus der die kristalline Phase dann mechanisch und/oder durch stufenweises Schmelzen abgetrennt werden kann.

Der angegebene Temperaturbereich 1 bis 25 °C unterhalb der konzentrationsabhängigen Liquidustemperatur ist unter wirtschaftlichen Aspekten bevorzugt, weil für jede unnötig tiefere Abkühlung zusätzliche Energie verbraucht würde. Für das dann erforderliche Erwärmen auf 1 bis 25 °C unterhalb der konzentrationsabhängigen Liquidustemperatur träfe das Gleiche zu.

Im Rahmen von Schritt (c) kann beispielsweise eine Abtrennung mechanisch erfolgen und/oder durch komplettes Abschmelzen nach Abtrennen der vorherigen Mutterlauge. Bevorzugt wird so gearbeitet, dass die Essigsäure vollständig kristallisiert wird, dann der gebildete Block wieder angeschmolzen und die Mutterlauge abgenommen wird. Der restliche Block wird dann vollständig geschmolzen und wiederum kristallisiert.

Schritt (c) des Verfahrens ist vorteilhaft, wenn es zu Einschlüssen der flüssigen Phase in der kristallinen Phase kommt und die beiden Phasen aufgrund der resultierenden hohen Viskosität nicht auf einfache Weise (z.B. durch Filtrieren) mechanisch voneinander getrennt werden können. Dies kann bei bestimmten Verhältnissen von kristalliner Phase zu flüssiger Phase der Fall sein. Als Faustregel reicht es in solchen Fällen aus, etwa 10 bis 20 Gew.-% der ursprünglich gebildeten kristallinen Phase durch graduelles Erwärmen wieder aufzuschmelzen, um die Viskosität so zu erniedrigen, dass eine mechanische Abtrennung (z.B. duch Filtrieren) möglich ist. Der Fachmann kann von dieser Faustregel gegebenfalls abweichende Aufschmelzbereiche problemlos durch Routineversuche ermitteln.

Das Abkühlen kann auf beliebige Weise erfolgen, beispielsweise durch Einstellen in einen Tiefkühlschrank oder mittels Kältemischungen (z.B. Trockeneis/Aceton bzw. Eis/Kochsalz) oder flüssigem Stickstoff.

Dem Fachmann ist klar, dass eine fraktionierte Kristallisation von Essigsäure auch aus der "entgegengesetzten Richtung" durch graduelles Abkühlen einer wässrigen Essigsäuremischung in Richtung der Liquidustemperatur erreicht werden kann. Dies ist aber unter praktischen und wirtschaftlichen Aspekten weniger vorteilhaft.

Die wässrige Essigsäuremischung kann mindestens 55, 60, 65, 70, 75, 80, 85, 90 oder 95 Gew.-% Essigsäure enthalten.

In Schritt (a) kann auf 1; 1,5; 2; 2,5, 3, 3,5; 4; 4,5, 5; 5,5; 6; 6,5; 7; 7,5; 8; 9; 10; 12; 15; 18; 20; 22, 25 °C unterhalb der konzentrationsabhängigen Liquidustemperatur der wässrigen Essigäuremischung abgekühlt werden.

In Schritt (a) kann 2 min bis 25 h abgekühlt werden. In Schritt (a) kann insbesondere 5 min, 10 min, 15 min, 20 min, 30 min, 40 min, 50 min, 60 min, 70 min, 80 min, 90 min, 2 h, 3 h, 4 h, 5 h, 6 h, 7 h, 8 h, 9 h, 10 h, 11 h, 12 h, 13 h, 14 h, 15 h, 16 h, 17 h, 18 h, 19 h, 20 h, 21 h, 22 h, 23 h oder 24 h abgekühlt werden.

Das mechanische Verfahren zum Abtrennen der gebildeten kristallinen Phase kann unter einem oder mehreren der Trennverfahren Filtration, Vakuumfiltration, Sedimentation, Zentrifugation und Fliehkraftabscheidung ausgewählt werden.

Im Anschluss an Schritt (b) oder (c) können gemäß einem Schritt (d) die Schritte (a) und (b) bzw. (a) und (c) mindestens einmal wiederholt werden.

Wenn das Verfahren wiederholt durchgeführt wird, kann nach Schritt a) jeweils ausschließlich Schritt b) oder (alternativ) ausschließlich Schritt c) durchgeführt werden. Es ist aber auch möglich, dass nach einer vorgegebenen Anzahl von Wiederholungen von Schritt b) auf Schritt c) (einmalig) gewechselt wird, oder umgekehrt. Es ist zudem möglich, dass z. B. nach einer Ausführung des Schritts a) das Kristallisationsergebnis bewertet und dann (bedarfsweise) entschieden wird, ob Schritt b) oder Schritt c) ausgeführt wird.

Im Folgenden wird die Erfindung detailliert beschrieben, wobei die offenbarten konkreten Ausführungsformen der Erfindung, Beispiele oder Ergebnisse lediglich zur Veranschaulichung dienen sollen und keinesfalls als Einschränkung des Schutzbereichs der Erfindung, wie dieser in den beigefügten Ansprüchen definiert ist, zu interpretieren sind.

Sofern nicht anders definiert, haben alle hier verwendeten technischen und wissenschaftlichen Begriffe die gleiche Bedeutung, wie sie von einem Fachmann auf dem technischen Gebiet der Erfindung verstanden werden. Dabei kann der Fachmann auch auf die einleitenden Erläuterungen vollumfänglich Bezug nehmen.

Die Verwendung von bestimmten bzw. unbestimmten Artikeln ("der", "die", "das", "ein", "eine"), ist (insbesondere im Zusammenhang mit den Ansprüchen) so zu verstehen, dass davon mindestens ein Element bzw. eine Komponente umfasst sein soll, sofern hier nichts anderes angegeben ist oder sich durch den Kontext eindeutig etwas anderes ergibt.

Der Konjunktion "oder" ist als einschließendes und nicht als ausschließendes "oder" zu verstehen, d. h. als "und/oder", sofern hier nichts anderes angegeben ist oder sich durch den Kontext eindeutig etwas anderes ergibt.

Die Verwendung von Begriffen wie "zum Beispiel", "z. B.", "wie" oder Abwandlungen davon soll lediglich zur besseren Veranschaulichung der Erfindung dienen und darf keinesfalls als Einschränkung des Schutzbereichs der Erfindung, wie er in den beigefügten Ansprüchen definiert ist, interpretiert werden.

Sämtliche numerischen Werte verstehen sich, unabhängig davon, ob dies ausdrücklich angegeben ist oder nicht, als "ungefähre" Werte (zumindest im Rahmen der üblichen Fehlertoleranzen). Die Angabe von Wertebereichen dient zudem lediglich zur Abkürzung und bezieht sich, sofern hier nicht anderes angegeben ist, auf jeden einzelnen Wert, der in den Bereich fällt, auch wenn dieser Wert nicht individuell angegeben wird.

Es ist bekannt, dass Essigsäure bei 16 - 17 °C fest wird. Dies führt in der industriellen Handhabung generell zu Problemen. Es wurde festgestellt, dass eine Mischung, die als Hauptkomponente Essigsäure und als Nebenkomponenten Wasser, Ameisensäure, Propionsäure, Buttersäure und andere Bestandteile enthält, bei einer Temperatur unterhalb des Schmelzpunktes von Essigsäure teilweise gefriert. Die Nebenkomponenten stören die Kristallisation der Essigsäure und erschweren dadurch deren Reinigung. Die Konzentrationen in der festen und der flüssigen Phase sind unterschiedlich. Die kristalline Phase ist an Essigsäure angereichert und an allen Begleitkomponenten Wasser, Ameisensäure, Propionsäure und Buttersäure abgereichert. In Anbetracht der sehr unterschiedlichen Schmelzpunkte und der chemischen Neigung von niedermolekularen organischen Säuren zur Bildung von Dimeren durch Wasserstoffbrücken ist die hohe Anreicherung von Essigsäure in Anwesenheit von Leicht- und Schwersiedern mittels einer Kristallisationstechnologie erstaunlich. Es wäre zu erwarten, dass Substanzen mit höherem Schmelzpunkt bei der Kristallisation auch bevorzugt ausfallen. Zudem arbeitet die Dimerenbildung gegen die Kristallisation. Je mehr Dimer, umso niedriger der Schmelzpunkt und umso höher die zur Kristallisation erforderliche Unterkühlung. Für die effektive Trennung ist eine genaue Verfahrensführung notwendig.

Ein Verfahren zur Aufreinigung von Essigsäure mittels fraktionierender Kristallisation aus wässrigen Essigsäuremischungen, die als Nebenbestandteile Ameisensäure, Propionsäure und Buttersäure enthalten, ist bislang nicht beschrieben. Kurzkettige Carbonsäuren bilden untereinander durch Wasserstoffbrückenbindungen zwischen den Carboxylgruppen Dimere. Das Kristallisationsverhalten eines Gemisches, das kurzkettige Carbonsäuren enthält (z.B. eines Ameisensäure/Essigsäure/Propionsäure/Buttersäure-Gemisches), ist wegen dieser Dimerbildung und weil zudem die Schmelz- bzw. Liquidustemperatur solcher Gemische von den Konzentrationen der einzelnen Carbonsäure-Komponenten abhängig ist, nicht vorhersagbar. Vor diesem Hintergrund ist zu erwarten, dass eine fraktionierende Kristallisation zur Trennung misslingen wird.

Es ist daher umso überraschender, dass mit dem hier vorgeschlagenen Verfahren gemäß Anspruch 1 eine solche fraktionierende Kristallisation zur Trennung möglich ist.

Vorteilhafte und/oder bevorzugte Ausführungsformen des hier vorgeschlagenen Verfahrens sind Gegenstand der Unteransprüche.

Um das erfindungsgemäße Verfahren auszuführen, ist eine Unterkühlung bezogen auf den Schmelzpunkt bzw. Liquiduspunkt von Essigsäure notwendig.

Der Grad der zur Kristallisation notwendigen Unterkühlung ist dabei abhängig von den Anteilen an Nebenkomponenten. Die Dauer der Unterkühlung beeinflusst die Ausbeute an der kristallinen Phase. Es wurde weiterhin festgestellt, dass es ab einem bestimmten Verhältnis von kristalliner Phase zu Flüssigkeit zu Einschlüssen der Flüssigkeit in der kristallinen Phase kommt und die beiden Phasen nicht voneinander getrennt werden können. Das Gemisch muss dann mechanisch gerührt werden oder die Temperatur muss so weit erhöht werden, dass eine aus der Kristallphase fließfähige Flüssigkeit entsteht.

Vorteilhafterweise wird erfindungsgemäß eine Essigsäuremischung als Ausgangsmaterial in dem erfindungsgemäßen Verfahren eingesetzt, die 70 bis 90 Gew.-% Essigsäure, 1 bis 5 Gew.-% Ameisensäure, 1 bis 5 Gew.-% Wasser, 1 bis 5 Gew.-% Propionsäure und 1 bis 5 Gew.-% Buttersäure enthalten kann. Dem Fachmann ist klar, dass sich die Gew.-%-Anteile der enthaltenen Komponenten auf 100 Gew.-% addieren. Konkrete Einzelwerte innerhalb der genannten Bereiche für die Komponenten der eingesetzten Essigsäuremischung sind 75, 80, 85 Gew.-% Essigsäure; 2, 3, 4 Gew.-% Ameisensäure; 2, 3, 4 Gew.-% Propionsäure und 2, 3, 4 Gew.-% Buttersäure. Selbstverständlich können je nach eingesetztem Ausgangsmaterial einzelne Komponenten auch gänzlich fehlen, als typisches Beispiel sei eine Essigsäuremischung genannt, die 90 Gew.-% Essigsäure, 5 Gew.-% Ameisensäure, 4 Gew.-% Wasser und 1 Gew.-% Buttersäure enthält.

Die Vorteile dieses erfindungsgemäßen Verfahrens sind der Verzicht auf Lösungsmittel, das Arbeiten bei niedrigen Temperaturen sowie die Abreicherung von sowohl Schwersiedern als auch Leichtsiedern in einem Verfahrensschritt.

In den folgenden Beispielen wird das erfindungsgemäße Verfahren näher erläutert. Diese Beispiele sollen nur zur Veranschaulichung der Erfindung dienen und dürfen keinesfalls als Einschränkung des Schutzbereichs der Erfindung, wie er in den beigefügten Ansprüchen definiert ist, interpretiert werden.

### Beispiel 1:

Der Grad der für die Kristallisation benötigten Unterkühlung ist wie folgt abhängig von den Konzentrationen an Essigsäure, Wasser, Ameisensäure, Propionsäure und Buttersäure. Eine Essigsäuremischung wird dazu auf -17 °C temperiert. Die Temperatur wird in der Essigsäuremischung gemessen. Sie fällt zunächst exponentiell und steigt bei Eintreten der Kristallisation auf einen Wert *T_{Sm}* (Schmelz- bzw. Liquidustemperatur) an und bleibt dann für eine bestimmte Zeit t konstant, bis sie schließlich wieder abnimmt. In der Tabelle 1 sind die Temperaturen *T_{Sm}* in Abhängigkeit einiger Essigsäuremischungen angegeben:

**Tabelle 1:**

| **Versuc h** | **Essigsäur e** | **Ameisensäu re** | **Wasse r** | **Propionsäu re** | **Buttersäur e** | **Andere*** | ***T_{Sm}* in °C** |
|---|---|---|---|---|---|---|---|
| | **in Gew.-%** | | | | | | |
| 1 | 100,0 | 0 | 0 | 0 | 0 | 0 | 16,3 |
| 2 | 96,0 | 4,0 | 0 | 0 | 0 | 0 | 13,1 |
| 3 | 92,0 | 8,0 | 0 | 0 | 0 | 0 | 9,4 |
| 4 | 88,0 | 12,0 | 0 | 0 | 0 | 0 | 5,6 |
| 5 | 84,0 | 16,0 | 0 | 0 | 0 | 0 | 2,7 |
| 6 | 80,0 | 20,0 | 0 | 0 | 0 | 0 | -0,8 |
| 7 | 86,1 | 4,4 | 3,7 | 4,2 | 0,4 | 1,2 | 4 |

| | | | | | | | |
|---|---|---|---|---|---|---|---|
| * beliebige andere Bestandteile, z.B. Aldehyde und Ketone. | | | | | | | |

### Beispiel 2:

Die Essigsäuremischung aus Versuch 7 in Beispiel 1 wird in ein Kältebad gestellt. Die Temperatur wird in der Essigsäuremischung gemessen. Die Temperatur des Kältebades wird periodisch kälter gestellt. Bei -1 °C in der Essigsäuremischung setzt die Kristallisation ein, die Temperatur steigt im weiteren Verlauf der Kristallisationsreaktion auf 4 °C.

### Beispiel 3:

Das Kristallwachstum hängt von der Zeit ab. Um die Abhängigkeit zu untersuchen, wird eine Essigsäuremischung mit 86,1 Gew.-% Essigsäure, 4,4 Gew.-% Ameisensäure, 3,7 Gew.-% Wasser, 4,2 Gew.-% Propionsäure, 0,4 Gew.-% Buttersäure in ein Becherglas überführt und in ein Kältebad mit einer Temperatur von -7 °C gestellt (Schmelz- bzw. Liquidustemperatur dieses Carbonsäure-Gemisches gemäß Tabelle 1: 4 °C). Nach Eintreten der Kristallisation, die durch eine Trübung erkennbar ist, wird 2 Minuten gewartet und anschließend über eine Nutsche filtriert. Die Fraktionen werden gewogen. Dieser Versuch wird für verschiedene Zeiten *t_{Kr}* (Kristallisationszeitdauer) wiederholt. In der Tabelle 2 sind die Resultate dargestellt:

**Tabelle 2:**

| **Versuch** | ***t_{Kr}* in min** | **Ausbeute Kristall in Gew.-%** |
|---|---|---|
| 1 | 2 | 9 |
| 2 | 10 | 15 |
| 3 | 90 | 31 |
| 4 | 1080 | 67 |

### Beispiel 4:

Ist das Verhältnis kristalline Phase zu Flüssigkeit zu hoch, kommt es zu Einschlüssen der Flüssigkeit und sie kann nicht mehr fließen. Die Mischung aus den Versuchen 1 und 2 aus Beispiel 3 können ohne weitere Aufarbeitung durch Ausschütten des Becherglases filtriert werden. Bei den Versuchen 3 und 4 aus Beispiel 2 entsteht eine nicht fließfähige Suspension. Es wird daher vor dem Ausschütten mit Hilfe eines Glasstabes mechanisch gerührt.

### Beispiel 5:

Die nicht fließfähige Suspension von Versuch 4 aus Beispiel 3 wird von -7 °C auf 2 °C erwärmt. Es entsteht eine fließfähige Suspension, die einfach durch Ausschütten filtriert werden kann.

### Beispiel 6:

Das Verfahren der Kristallisation wird hier mehrstufig angewendet. Es werden 60 g einer Essigsäuremischung, die 86,1 Gew.-% Essigsäure, 4,4 Gew.-% Ameisensäure, 3,7 Gew.-% Wasser, 4,2 Gew.-% Propionsäure und 0,4 Gew.-% Buttersäure enthält, in ein Becherglas überführt. In einer Kristallisationsstufe 1 wird die Mischung für 30 Minuten bei -18 °C gehalten. Das Becherglas wird mit der Öffnung nach unten hin auf einen Filter gestellt und auf 2 °C erwärmt. Wenn keine Tröpfchenbildung am Auslass des Filters mehr zu erkennen ist, wird das Filtrat gewogen und ein Gaschromatogramm aufgenommen. In einer Kristallisationsstufe 2 wird von 2 °C auf 7 °C erwärmt. Es wird weiter vorgegangen wie oben beschrieben. In einer Kristallisationsstufe 3 wird von 7 °C auf 9 °C erwärmt und wie oben beschrieben weiter verfahren. Die Bestimmung der Konzentration in der kristallinen Phase erfolgt rechnerisch über die Massenbilanz. In der Tabelle 3 sind die Resultate dieser mehrstufigen Kristallisation dargestellt:

**Tabelle 3:**

| | **Ausbeute in g** | **Essigsäu re** | **Ameisensäure** | **Wass er** | **Propionsäure** | **Buttersäure** | **Ander e** |
|---|---|---|---|---|---|---|---|
| | | **in Gew.-%** | | | | | |
| **Essigsäuremischu ng** | 60,0 | 86,1 | 4,4 | 3,7 | 4,2 | 0,4 | 1,2 |
| **Kristall Stufe 1** | 32,6 | 89,0 | 3,4 | 3,1 | 3,1 | 0,3 | 1,1 |
| **Kristall Stufe 2** | 22,2 | 91,4 | 2,7 | 2,3 | 2,3 | 0,2 | 1,1 |
| **Kristall Stufe 3** | 15,4 | 93,7 | 2,1 | 1,7 | 1,9 | 0,1 | 0,5 |

Fachleuten auf dem technischen Gebiet der Erfindung ist klar, dass die oben beschriebenen repräsentativen Ausführungsformen und Details der Erfindung nur zur Veranschaulichung der vorliegenden Erfindung dienen sollen, und dass verschiedene Abänderungen und Modifikationen vorgenommen werden können, ohne dadurch den Schutzbereichs der Erfindung, wie er in den beigefügten Ansprüchen definiert ist, zu verlassen.

## Patentansprüche

1. Verfahren zur Anreicherung von Essigsäure aus wässrigen Essigsäuremischungen, die mindestens 50 Gew.-% Essigsäure und Nebenbestandteile ausgewählt aus der aus Ameisensäure, Propionsäure und Buttersäure bestehenden Gruppe enthalten, **dadurch gekennzeichnet, dass**
(a) eine wässrige Essigsäuremischung durch Abkühlen auf eine Temperatur von 1 bis 25 °C unterhalb der konzentrationsabhängigen Liquidustemperatur kristallisiert wird, und
(b) die gebildete kristalline Phase von der flüssigen Phase mechanisch abgetrennt wird, oder
(c) die gebildete kristalline Phase durch graduelles Erwärmen zum Teil wieder aufgeschmolzen und die verbleibende kristalline Phase anschließend mechanisch abgetrennt wird.

2. Verfahren nach Anspruch 1, **dadurch gekennzeichnet, dass** die wässrige Essigsäuremischung mindestens 55, 60, 65, 70, 75, 80, 85, 90 oder 95 Gew.-% Essigsäure enthält.

3. Verfahren nach Anspruch 1 oder 2, **dadurch gekennzeichnet, dass** in der wässrigen Essigsäuremischung 1 bis 5 Gew.-% Ameisensäure und/oder 1 bis 5 Gew.-% Propionsäure und/oder 1 bis 5 Gew.-% Buttersäure enthalten sind.

4. Verfahren nach einem der Ansprüche 1 bis 3, **dadurch gekennzeichnet, dass** in Schritt (a) auf 1; 1,5; 2; 2,5, 3, 3,5; 4; 4,5, 5; 5,5; 6; 6,5; 7; 7,5; 8; 9; 10; 12; 15; 18; 20; 22, 25 °C unterhalb der konzentrationsabhängigen Liquidustemperatur der wässrigen Essigsäuremischung abgekühlt wird.

5. Verfahren nach einem der Ansprüche 1 bis 4, **dadurch gekennzeichnet, dass** in Schritt (a) 2 min bis 25 h abgekühlt wird.

6. Verfahren nach Anspruch 5, **dadurch gekennzeichnet**, das in Schritt (a) 5 min, 10 min, 15 min, 20 min, 30 min, 40 min, 50 min, 60 min, 70 min, 80 min, 90 min, 2 h, 3 h, 4 h, 5 h, 6 h, 7 h, 8 h, 9 h, 10 h, 11 h, 12h, 13 h, 14h, 15 h, 16h, 17 h, 18 h, 19 h, 20 h, 21 h, 22 h, 23 h oder 24 h abgekühlt wird.

7. Verfahren nach einem der Ansprüche 1 bis 6, **dadurch gekennzeichnet, dass** das mechanische Verfahren zum Abtrennen der gebildeten kristallinen Phase unter einem oder mehreren der Trennverfahren Filtration, Vakuumfiltration, Sedimentation, Zentrifugation und Fliehkraftabscheidung ausgewählt wird.

8. Verfahren nach einem der Ansprüche 1 bis 7, **dadurch gekennzeichnet, dass** dieses mehrstufig durchgeführt wird, wobei im Anschluss an Schritt (b) oder (c) (d) die Schritte (a) und (b) bzw. (a) und (c) mindestens einmal wiederholt werden.
